# EUROPÄISCHE PATENTSCHRIFT

(11) **EP 0 672 830 B1**
(45) Veröffentlichungstag und Bekanntmachung des Hinweises auf die Patenterteilung: **06.05.1998**
(21) Anmeldenummer: 94102333.5
(22) Anmeldetag: 16.02.1994
(51) Int. Cl.: F04B 43/12, F04B 43/00

(54) **Schlauchfixierungsvorrichtung für Rollenpumpen**
Tube fixation device for roller pumps
Dispositif de fixation du tube pour pompes à galets

(43) Veröffentlichungstag der Anmeldung: 20.09.1995
(73) Patentinhaber: Stöckert Instrumente GmbH, D-80939 München (DE)
(72) Erfinder: Friedmann, Günter Dr.-Ing, D-83236 Uebersee (DE); Wiehan, Helmut, D-85354 Freising (DE); Knott, Erwin Dr.-Ing, D-85586 Poing (DE)
(74) Vertreter: Zangs, Rainer E., Dipl.-Ing.

(56) Entgegenhaltungen:
- WO-A-93/22560
- DE-A- 3 326 784
- FR-A- 2 594 496
- GB-A- 1 119 149
- US-A- 4 585 399
- US-A- 4 798 590
- US-A- 4 976 590

## Beschreibung

Die Erfindung betrifft eine Schlauchfixierungsvorrichtung an einer Rollenpumpe einer Herz-Lungen-Maschine, die einen aus Pumpenstator und Pumpenrotor bestehenden Pumpenkopf aufweist, mit Fixierungseinrichtungen zur Fixierung eines in den Pumpenkopf der Rollenpumpe eingelegten Schlauchstücks.

Rollenpumpen, wie sie beispielsweise aus DE-A-33 26 784 und US-A-4 976 590 bekannt sind, werden in der Medizintechnik, insbesondere in Herz-Lungen-Maschinen zum Fördern von Blut in einem künstlichen Kreislauf eingesetzt. Grundsätzlich bestehen Rollenpumpen aus einem Pumpenkopf und einem Pumpenantrieb, von denen wiederum der Pumpenkopf aus einem Pumpenstator und einem Pumpenrotor besteht. Der Pumpenstator ist ein im wesentlichen zylindrischer Hohlraum, dessen als Pumpenbett bezeichnete Innenwand als Lager für ein in den Pumpenkopf eingelegtes, an der Innenwand anliegendes Schlauchstück dient. Der um seine Mittellängsachse drehbare Pumpenrotor ist in dem Pumpenstator so angeordnet, daß an einem Rollenträger drehbar gelagerte Rollen an dem Schlauchstück abrollbar sind und dabei das Schlauchstück zusammendrücken. Der Pumpenstator besitzt für das Herausführen des Schlauches zumindest einen geöffneten Abschnitt, durch den das Schlauchstück aus dem inneren Hohlraum des Pumpenstators geführt wird. Um zu vermeiden, daß das Schlauchstück unter der Einwirkung der auf ihm abrollenden Rollen des Pumpenrotors wandert, muß zumindest ein Ende des Schlauchstücks am Pumpenstator fixiert werden. Bei Rollenpumpen mit umkehrbarer Laufrichtung ist eine Fixierung an beiden Enden des Schlauchstücks erforderlich.

Bekannte Rollenpumpen besitzen aufgrund dieser Anforderung Fixierungseinrichtungen unterschiedlichster Bauform, beispielsweise Klemmelemente, die in den Pumpenstator integriert sind und eine sichere Fixierung des Schlauchstücks gewährleisten.

Davon ausgehend liegt der Erfindung die Aufgabe zugrunde, eine Verbesserung im Bereich der Schlauchfixierung von Rollenpumpen, insbesondere für Herz-Lungen-Maschinen aufzuzeigen, durch die insbesondere eine Flexibilisierung der Schlauchfixierung erreicht werden soll.

Gelöst wird diese Aufgabe durch eine Schlauchfixierungsvorrichtung mit den Merkmalen des Patentanspruchs 1.

Die Erfindung beruht auf der Erkenntnis, daß sich der Pumpenstator in zwei sich aufgrund ihrer Funktion deutlich unterscheidende Teile aufteilen läßt, nämlich das die Eigenschaften der Pumpfunktion definierende Pumpenbett und die eine Haltefunktion bereitstellende Schlauchfixierung. Durch die Einführung eines dieser gedanklichen Trennung entsprechenden Schlauchfixierungsmoduls, das als Einheit vom Pumpenkopf der Rollenpumpe lösbar ist, werden mehrere Vorteile erzielt.

Zum einen kann durch verschiedene Schlauchfixierungsmodule ein und derselbe Pumpenkopf mit unterschiedlichen Haltesystemen für das Schlauchstück bis hin zur integral verbundenen Ausführung ausgestattet werden. Zum anderen können Sensoren in der Schlauchfixierungseinheit angeordnet oder eingebettet werden, was die Möglichkeit eröffnet, einen defekten Sensor durch Auswechseln der Schlauchfixierungsvorrichtung auszutauschen.

Um die erfindungsgemäße Schlauchfixierungsvorrichtung am Pumpenstator zu befestigen, sind im Schlauchfixierungsmodul oder im Pumpenstator der Rollenpumpe Nuten vorgesehen, in die Vorsprünge einführbar und darin verschiebbar sind, die am Pumpenstator der Rollenpumpe bzw. am Schlauchfixierungsmodul ausgebildet sind.

Bei den im Schlauchfixierungsmodul vorgesehenen Sensoren kann es sich um Sensoren zur Messung von Eigenschaften des von der Rollenpumpe geförderten Mediums handeln, die in die Schlauchfixierungsvorrichtung integriert, beispielsweise in dafür vorgesehene Aufnahmen eingesetzt oder in das Material eingebettet sind. Als Sensoren bieten sich, insbesondere bei Herz-Lungen-Maschinen Durchflußmengensensoren, Strömungsgeschwindigkeitssensoren und Blasendetektoren an.

Im Schlauchfixierungsmodul sind die Fixierungseinrichtungen zur Fixierung des in dem Pumpenkopf der Rollenpumpe eingelegten Schlauchstücks vorgesehen. Dabei kann es sich um innen-konische Aufnahmen für außen-konische Klemmelemente handeln. Die Klemmelemente besitzen eine Öffnung für die Durchführung des Schlauches, deren Durchmesser geringfügig kleiner als der Durchmesser des Schlauches ist. Die Klemmelemente bestehen im wesentlichen aus zwei Teilen, die miteinander so verbunden sind, daß durch das Einstecken des Klemmelements in die Aufnahme der Schlauch aufgrund des Zusammenwirkens des Innenkonus der Aufnahme und des Außenkonus des Klemmelements in der Öffnung des Klemmelements eingeklemmt wird. Die beiden Teile des Klemmelements können mittels einer scharnierartigen Einrichtung verbunden sein, die an dem Klemmelement so angeordnet ist, daß sie mit einer in der Aufnahme vorgesehenen Führungsnut zur Ausrichtung des Klemmelements zusammenwirkt. Alternativ kann ein Filmscharnier die beiden Teile des Klemmelements verbinden, so daß es sich letztlich um eine einstückige Ausgestaltung handelt. In der Aufnahme und an dem Klemmelement kann eine Rasteinrichtung ausgebildet sein, die beim Einstecken des Klemmelements in die Aufnahme einrastbar ist. Dabei handelt es sich vorzugsweise um eine in der Innenwand der Aufnahme umlaufende Nut, in die eine aus der Außenwand des Klemmelements hervorspringende, umlaufende Wulst eingreift.

In einer abgewandelten Ausgestaltung können im Schlauchfixierungsmodul Aufnahmen vorgesehen sein, die an Fixierkörpern angepaßt sind, die am Schlauchstück angebracht sind. Die Fixierkörper können am Schlauchstück angeformt sein und aus dem gleichen Material wie das Schlauchstück bestehen.

In einer weiteren Ausgestaltung ist das Schlauchstück fest mit dem Schlauchfixierungsmodul verbunden und vorzugsweise verklebt oder umspritzt.

Im folgenden wird die Erfindung anhand eines Ausführungsbeispiels unter Bezugnahme auf die beiliegenden Zeichnungen genauer beschreiben. Darin zeigt:
- Fig. 1: ein Ausführungsbeispiel einer erfindungsgemäßen Schlauchfixierungsvorrichtung an einem Pumpenkopf einer Rollenpumpe für eine Herz-Lungen-Maschine;
- Fig. 2A-2C: das Ausführungsbeispiel der Schlauchfixierungsvorrichtung aus Fig. 1 in drei verschiedenen Ansichten; und
- Fig. 3: ein Ausführungsbeispiel eines Klemmelements für die Schlauchfixierungsvorrichtung gemäß Fig. 1 bzw. 2A - 2C.

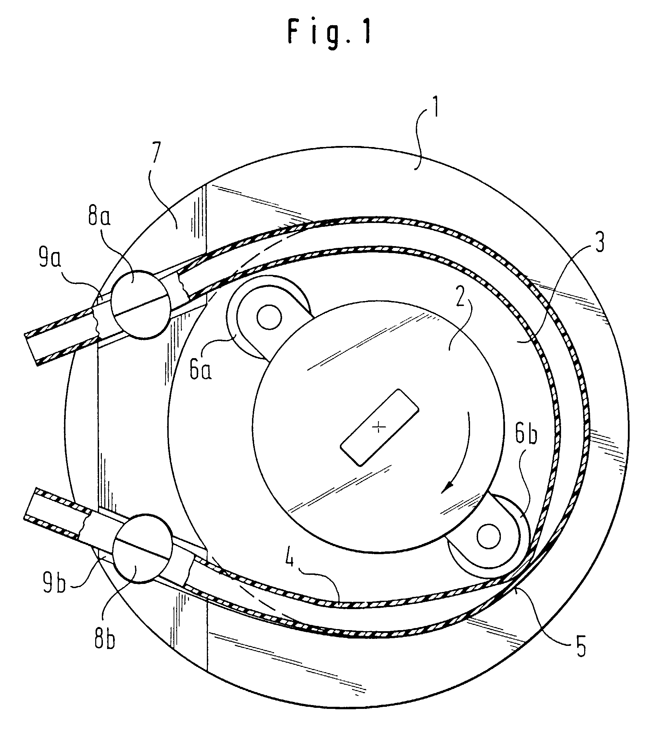

In Fig. 1 ist eine Rollenpumpe dargestellt, die aus einem Pumpenstator 1 und einem Pumpenrotor 2 besteht. In den im wesentlichen zylindrischen Hohlraum 3 des Pumpenstators ist ein Schlauch 4 so eingelegt, daß er an der als Pumpenbett bezeichneten Innenwand 5 des Pumpenstators anliegt. Die drehbar gelagerten Rollen 6a und 6b des Pumpenrotors rollen auf dem Schlauchstück ab und drücken es gegen die Innenwand 5 des Pumpenstators zusammen. Dadurch wird das im Schlauchstück vorhandene Medium entsprechend der Drehrichtung (Pfeil) des Pumpenrotors gefördert.

An dem für das Herausführen des Schlauchstücks geöffneten Abschnitt des Pumpenstators 1 ist erfindungsgemäß eine Schlauchfixierungsvorrichtung 7 angeordnet, die vom Pumpenstator und damit vom Pumpenkopf der Rollenpumpe lösbar ist; d.h. die Schlauchfixierungsvorrichtung 7 ist zwar am Pumpenstator 1 befestigt, kann aber abgenommen werden. Zwei Fixierungseinrichtungen 8a und 8b sind in der Schlauchfixierungsvorrichtung 7 vorgesehen und legen die beiden aus dem Pumpenstator herausgeführten Enden des Schlauchstücks 4 fest.

Die mit der erfindungsgemäßen Schlauchfixierungsvorrichtung 7 ausgestattete Rollenpumpe unterscheidet sich hinsichtlich ihrer Verwendung nicht von herkömmlichen Rollenpumpen. Wie bisher kann das Schlauchstück in das Pumpenbett eingelegt und mit Hilfe der Fixierrichtungen 8a und 8b fixiert werden. Jedoch gestattet die lösbare Ausgestaltung der Schlauchfixierungsvorrichtung 7 einen Austausch allein dieses Moduls, so daß unterschiedliche Fixierungseinrichtungen 8a und 8b an ein und demselben Pumpenkopf zum Einsatz kommen können. Ferner können im Bereich der Durchtrittskanäle 9a und 9b Sensoren (nicht dargestellt) vorgesehen werden, die mit der Schlauchfixierungsvorrichtung 7 verbunden sind und mit ihr am Pumpenkopf angebracht werden. Die Sensoren sind vorzugsweise in Aufnahmen eingesteckt oder in die Schlauchfixierungsvorrichtung eingebettet. Insbesondere die zuletzt genannte Ausgestaltung ermöglicht eine vor Beschädigungen gesicherte und gleichzeitig lage-genaue Anordnung der Sensoren. Sollte es bei einem Sensor zu einem Defekt kommen, kann die Schlauchfixierungsvorrichtung problemlos vom Pumpenkopf getrennt und erneuert werden. Eine Einbettung von Sensoren im Pumpenstator der Rollenpumpe ist nicht praktikabel, da bei defektem Sensor die gesamte Rollenpumpe zerlegt werden müßte, um den Pumpenstator auszutauschen.

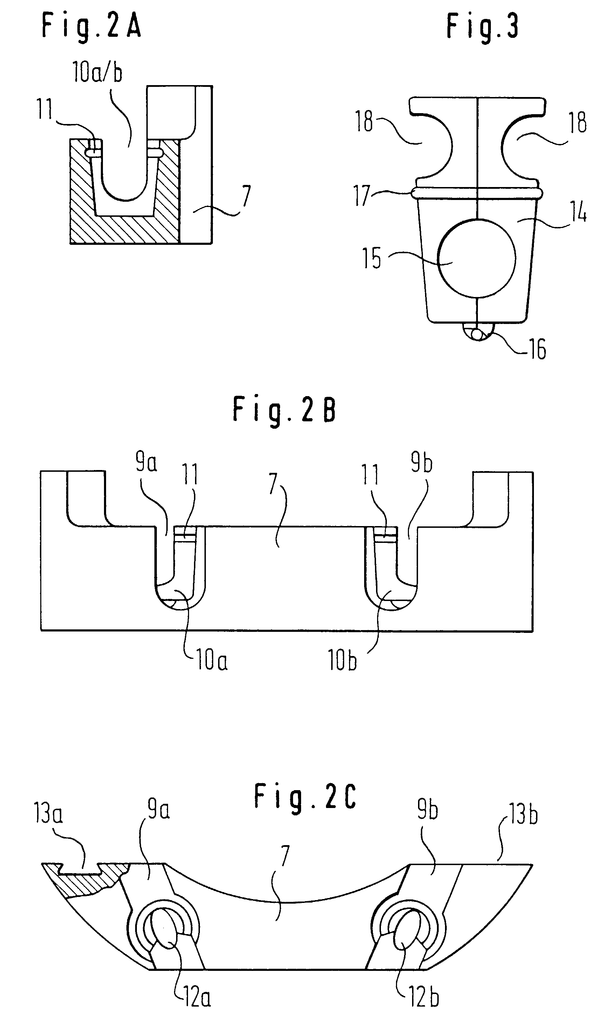

In den Fig. 2A, 2B und 2C ist die Schlauchfixierungsvorrichtung 7 aus Fig. 1 in unterschiedlichen Ansichten dargestellt. In den Fig. 2B und 2C erkennt man die beiden Kanäle 9a für die beiden Enden des Schlauchstücks. In diesen Kanälen sind Aufnahmen 10a für jeweils ein Klemmelement ausgestaltet. In Fig. 2A ist eine der Aufnahmen 10a/b in geschnittener Darstellung gezeigt. Man erkennt die innenkonische Ausgestaltung der Aufnahme und die in der Innenwand ausgebildete, umlaufende Nut 11, die Teil einer Rasteinrichtung darstellt, mit der das Klemmelement in der Aufnahme 10a/b einrastbar ist. Am Boden der Aufnahmen 10a/b sind Nuten 12a/b vorgesehen, die zur Ausrichtung des Klemmelements in der Aufnahme dienen, was im Zusammenhang mit dem Klemmelement noch genauer beschrieben wird.

Die Schlauchfixierungsvorrichtung 7 besitzt zwei Nuten 13a/b von denen eine Nut 13a in einer teilweise geschnittenen Darstellung in Fig. 2c dargestellt ist. Die Nuten 13a/b dienen zur Befestigung der Schlauchfixierungsvorrichtung 7 am Pumpenstator 1 (Fig. 1). Am Pumpenstator sind entsprechend geformte Vorsprünge vorgesehen, die in die Nuten 13a/b der Schlauchfixierungsvorrichtung einführbar und darin verschiebbar sind. Die Schlauchfixierungsvorrichtung 7 wird von oben auf die Vorsprünge aufgesteckt und auf den Pumpenstator abgesenkt, wobei die Vorsprünge in den Nuten 13a/b gleiten.

In Fig. 3 ist ein Klemmelement 14 gezeigt, das in eine der beiden Aufnahmen 9a/b (Fig. 2A-2C) eingesetzt werden kann. Das Klemmelement besitzt eine Öffnung 15 mit einem Durchmesser, die ein wenig kleiner als der Durchmesser des Schlauchstücks ist, das in die Rollenpumpe eingelegt wird. Um das Schlauchstück in die Öffnung 15 des Klemmelements 14 einlegen zu können, ist das Klemmelement in zwei Teile geteilt, die über ein Scharnier 16 an der Unterseite des Klemmelements miteinander schwenkbar verbunden sind. Das Scharnier 16 ist an die Nut 12a/b (Fig. 2C) am Boden der Aufnahmen so angepaßt, daß eine Ausrichtung des Klemmelements in der Aufnahme 9a/b durch Zusammenwirken des Scharniers 16 und der Nut 12a/b erfolgt.

Oberhalb der Durchtrittsöffnung 15 ist eine umlaufende Wulst 17 vorgesehen, die als Gegenstück zur umlaufenden Nut 11 in der Aufnahme 10a/b (Fig. 2A) den zweiten Teil der Rasteinrichtung darstellt. Wird das Klemmelement 14 mit dem in die Druchtrittsöffnung 15 eingelegten Schlauch in eine der Aufnahmen 9a/b eingesteckt, wird aufgrund der konischen Form des Klemmelements und der Aufnahme das Schlauchstück in der Durchtrittsöffnung 15 eingeklemmt. Die Wulst 17 des Klemmelements rastet in die Nut 11; der Aufnahme ein, gleichzeitig wird das Klemmelement durch das Zusammenspiel des Scharniers 16 und der Nut 12a/b ausgerichtet.

Vorzugsweise besitzt das Klemmelement einen Griffbereich 18, der ein Einsetzen und Herausziehen des Klemmelements vereinfacht.

Die Schlauchfixierungsvorrichtung kann in einer abgewandelten Ausführungsform mit Aufnahmen ausgerüstet werden, in die Fixierkörper einsetzbar sind, die am Schlauchstück selbst angeformt sind. Darüber hinaus kann die Fixierung alternativ auch dadurch erfolgen, daß das Schlauchstück fest mit der Schlauchfixierungsvorrichtung verbunden ist, in der es in geeignet ausgestalteten Kanälen, entsprechend den in Fig. 2B und 2C gezeigten Kanälen 9a/b der Schlauchfixierungsvorrichtung verklebt oder umspritzt wird.

## Patentansprüche

1. Schlauchfixierungsvorrichtung an einer Rollenpumpe einer Herz-Lungen-Maschine, die einen aus Pumpenstator und Pumpenrotor bestehenden Pumpenkopf aufweist, mit Fixierungseinrichtungen zur Fixierung eines in den Pumpenkopf der Rollenpumpe eingelegten Schlauchstücks,
**dadurch gekennzeichnet, daß** beide Enden des Schlauchstück (4) durch die Schlauchfixierungsvorrichtung (7) aus dem Pumpenkopf herausgeführt sind und die Schlauchfixierungsvorrichtung (7) zusammen mit dem fixierten Schlauchstück (4) vom Pumpenkopf (1,2) der Rollenpumpe lösbar ist.

2. Schlauchfixierungsvorrichtung nach Anspruch 1, dadurch **gekennzeichnet,** daß Nuten (13a/b) vorgesehen sind, in die zur Befestigung der Schlauchfixierungsvorrichtung (7) an dem Pumpenkopf (1,2) der Rollenpumpe Vorsprünge einführbar und darin verschiebbar sind, die an dem Pumpenstator (1) der Rollenpumpe ausgebildet sind.

3. Schlauchfixierungsvorrichtung nach Anspruch 1, dadurch **gekennzeichnet,** daß Vorsprünge vorhanden sind, die zur Befestigung der Schlauchfixierungsvorrichtung (7) an dem Pumpenkopf (1, 2) der Rollenpumpe in Nuten einführbar und darin verschiebbar sind, die an dem Pumpenstator (11) der Rollenpumpe ausgebildet sind.

4. Schlauchfixierungsvorrichtung nach Anspruch 1, 2 oder 3, dadurch **gekennzeichnet**, daß Sensoren zur Messung von Eigenschaften des von der Rollenpumpe geförderten Mediums in die Schlauchfixierungsvorrichtung (7) integriert sind.

5. Schlauchfixierungsvorrichtung nach Anspruch 4, dadurch **gekennzeichnet,** daß Aufnahmen für die Sensoren vorgesehen sind, in die die Sensoren einsetzbar sind.

6. Schlauchfixierungsvorrichtung nach Anspruch 4, dadurch **gekennzeichnet**, daß die Sensoren in das Material der Schlauchfixierungsvorrichtung (7) eingebettet sind.

7. Schlauchfixierungsvorrichtung nach einem der Ansprüche 1 bis 6, dadurch **gekennzeichnet,** daß innen-konische Aufnahmen (9a/b) für außen-konische Klemmelemente (14) vorgesehen sind, daß die Klemmelemente (14) aus im wesentlichen zwei Teilen bestehen und eine Öffnung (15) für die Durchführung eines Schlauches aufweisen, deren Durchmesser geringfügig kleiner als der Durchmesser des Schlauches ist, daß die zumindest beiden Teile der Klemmelemente (14) miteinander so verbunden sind, daß durch das Einstecken der Klemmelemente (14) in die Aufnahmen (9a/b) der Schlauch aufgrund des Zusammenwirkens des Innenkonus der Aufnahme und des Außenkonus des Klemmelements in der Öffnung (15) des Klemmelements eingeklemmt wird.

8. Schlauchfixierungsvorrichtung nach Anspruch 7, dadurch **gekennzeichnet,**daß die beiden Teile der Klemmelemente (14) mittels einer scharnierartigen Einrichtung (16) verbunden sind, die an den Klemmelementen (14) so angeordnet ist, daß sie mit in den Aufnahmen vorgesehenen Führungsnuten (12a/b) zur Ausrichtung des Klemmelements in der Aufnahme zusammenwirkt.

9. Schlauchfixierungsvorrichtung nach Anspruch 7 oder 8, dadurch **gekennzeichnet**, daß ein Filmscharnier vorgesehen ist, das die wesentlichen zwei Teile des Klemmelements (14) verbindet.

10. Schlauchfixierungsvorrichtung nach Anspruch 9, dadurch **gekennzeichnet**, daß das Filmscharnier einstückig mit den im wesentlichen zwei Teilen des Klemmelements (14) ausgebildet ist.

11. Schlauchfixierungsvorrichtung nach einem der Ansprüche 7 bis 10, dadurch **gekennzeichnet**, daß in den Aufnahmen (9a/b) und an den Klemmelementen (14) Rasteinrichtungen (11, 17) ausgebildet sind, die beim Einstecken der Klemmelemente in die Aufnahmen einrastbar sind.

12. Schlauchfixierungsvorrichtung nach einem der Ansprüche 1 bis 6, dadurch **gekennzeichnet**, daß Aufnahmen für Fixierkörper vorgesehen sind, die an dem Schlauchstück angebracht sind.

13. Schlauchfixierungsvorrichtung nach Anspruch 12, adurch **gekennzeichnet**, daß die Fixierkörper an dem Schlauchstück angeformt sind.

14. Schlauchfixierungsvorrichtung nach einem der Ansprüche 1 bis 6, dadurch **gekennzeichnet**, daß das Schlauchstück fest mit der Schlauchfixierungsvorrichtung verbunden ist.

15. Schlauchfixierungsvorrichtung nach Anspruch 14, dadurch **gekennzeichnet**, daß das Schlauchstück verklebt oder umspritzt ist.

16. Rollenpumpe für eine Herz-Lungen-Mschine mit einer Schlauchfixierungsvorrichtung (7) nach einem der vorangegangenen Ansprüche 1 bis 15.

## Claims

1. Hose-fixing device on a roller pump of a heart-lung machine, which has a pump head consisting of a pump stator and a pump rotor, with fixing devices for fixing a hose section inserted into the pump head of the roller pump,
characterised in that both ends of the hose section (4) are passed through the hose fixing device (7) out of the pump head and the hose fixing device (7) is detachable together with the fixed hose section (4) from the pump head (1,2) of the roller pump.

2. Hose fixing device according to claim 1, characterised in that grooves (13a/b) are provided, into which projections are introducible and are displaceable therein in order to secure the hose fixing device (7) to the pump head (1,2) of the roller pump, said projections being formed on the pump stator (1) of the roller pump.

3. Hose fixing device according to claim 1, characterised in that projections are present which are introducible, in order to secure the hose fixing device (7) to the pump head (1,2) of the roller pump, into grooves and are displaceable therein, said grooves being formed on the pump stator (11) of the roller pump.

4. Hose fixing device according to claim 1, 2 or 3, characterised in that sensors for measuring properties of the medium conveyed by the roller pump are integrated in the hose fixing device (7).

5. Hose fixing device according to claim 4, characterised in that receiving means are provided for the sensors, into which the sensors may be inserted.

6. Hose fixing device according to claim 4, characterised in that the sensors are embedded in the material of the hose fixing device (7).

7. Hose fixing device according to one of claims 1 to 6, characterised in that internally conical receiving means (9a/b) are provided for externally conical clamp members (14), in that the clamp members (14) substantially consist of two parts, and have an opening (15) for through passage of a hose, and whose diameter is slightly smaller than the diameter of the hose, in that the at least two parts of the clamp members (14) are connected together in such a way that, by means of inserting the clamp members (14) into the receiving means (9a/b), the hose is clamped in the opening (15) of the clamp member due to the co-operation of the internal cone of the receiving means and the external cone of the clamp member.

8. Hose fixing device according to claim 7, characterised in that the two parts of the clamp members (14) are connected by means of a hinge-like arrangement (16) which is disposed on the clamp members (14) in such a way that it co-operates with the guide grooves (12a/b) provided in the receiving means to align the clamp member in the receiving means.

9. Hose fixing device according to claim 7 or 8, characterised in that a film hinge is provided, which connects the substantially two parts of the clamp member (14).

10. Hose fixing device according to claim 9, characterised in that the film hinge is integrally formed as one piece with the substantially two parts of the clamp member (14).

11. Hose fixing device according to one of claims 7 to 10, characterised in that there are formed in the receiving means (9a/b) and on the clamp members (14) resilient engagement devices (11,17), which snap into the receiving means when the clamp members are inserted.

12. Hose fixing device according to one of claims 1 to 6, characterised in that receiving means are provided for fixing bodies which are attached to the hose section.

13. Hose fixing device according to claim 12, characterised in that the fixing bodies are integrally moulded on the hose section.

14. Hose fixing device according to one of claims 1 to 6, characterised in that the hose section is securely connected to the hose fixing device.

15. Hose fixing device according to claim 14, characterised in that the hose section is glued or extrusion-coated.

16. Roller pump for a heart-lung machine with a hose fixing device (7) according to one of the preceding claims 1 to 15.

## Revendications

1. Dispositif de fixation de tuyau souple dans une pompe à galets d'une machine cardio-pulmonaire qui comporte une tête de pompe constituée d'un stator de pompe et d'un rotor de pompe, le dispositif de fixation comprenant des moyens de fixation destinés à fixer une partie de tuyau souple placée dans la tête de pompe de la pompe à galets,
**caractérisé** en ce que les deux extrémités de la partie de tuyau souple (4) sortent hors de la tête de pompe par le dispositif de fixation de tuyau souple (7), et en ce que le dispositif de fixation de tuyau souple (7) peut être démonté en commun avec la partie de tuyau souple (4) fixée, de la tête de pompe (1, 2) de la pompe à galets.

2. Dispositif de fixation de tuyau souple selon la revendication 1, **caractérisé** en ce que sont prévues des rainures (13a/b) dans lesquelles peuvent être introduites et dans lesquelles peuvent coulisser des protubérances pour fixer le dispositif de fixation de tuyau souple (7) à la tête de pompe (1, 2), ces rainures étant formées dans le stator de pompe (1) de la pompe à galets.

3. Dispositif de fixation de tuyau souple selon la revendication 1, **caractérisé** en ce qu'il existe des protubérances qui peuvent être introduites et peuvent coulisser dans des rainures pour fixer le dispositif de fixation de tuyau souple (7) à la tête de pompe (1, 2) de la pompe à galets, ces protubérances étant réalisées sur le stator de pompe (1).

4. Dispositif de fixation de tuyau souple selon la revendication 1, 2 ou 3, **caractérisé** en ce que des capteurs destinés à mesurer des propriétés du fluide refoulé par la pompe à galets, sont intégrés dans le dispositif de fixation de tuyau souple (7).

5. Dispositif de fixation de tuyau souple selon la revendication 4, **caractérisé** en ce que sont prévus des logements pour les capteurs, dans lesquels peuvent être insérés ces capteurs.

6. Dispositif de fixation de tuyau souple selon la revendication 4, **caractérisé** en ce que les capteurs sont noyés dans le matériau du dispositif de fixation de tuyau souple (7).

7. Dispositif de fixation de tuyau souple selon l'une des revendications 1 à 6, **caractérisé** en ce que sont prévus des logements (9a/b) coniques intérieurs pour des éléments de serrage (14) coniques extérieurs, en ce que les éléments de serrage (14) sont constitués essentiellement de deux pièces et présentent une ouverture (15) pour le passage d'un tuyau souple et dont le diamètre est légèrement inférieur au diamètre du tuyau souple, en ce que lesdites au moins deux pièces des éléments de serrage (14) sont reliées l'une à l'autre de manière telle, que l'insertion des éléments de serrage (14) dans les logements (9a/b) produit le serrage du tuyau souple dans l'ouverture (15) de l'élément de serrage en raison de l'interaction du cône intérieur du logement et du cône extérieur de l'élément de serrage.

8. Dispositif de fixation de tuyau souple selon la revendication 7, **caractérisé** en ce que les deux pièces des éléments de serrage (14) sont reliées au moyen d'un mécanisme (16) du type à charnière, qui est placé sur les éléments de serrage (14) de façon à coopérer avec des rainures de guidage (12a/b) prévues dans les logements, en vue de l'orientation de l'élément de serrage dans le logement.

9. Dispositif de fixation de tuyau souple selon la revendication 7 ou 8, **caractérisé** en ce qu'il est prévu une charnière à film qui relie les deux pièces essentielles de l'élément de serrage (14).

10. Dispositif de fixation de tuyau souple selon la revendication 9, **caractérisé** en ce que la charnière à film est réalisée d'un seul tenant avec les deux pièces essentielles de l'élément de serrage (14).

11. Dispositif de fixation de tuyau souple selon l'une des revendications 7 à 10, **caractérisé** en ce que dans les logements (9a/b) et sur les éléments de serrage (14) sont réalisés des mécanismes d'encliquetage (11, 17), qui s'enclenchent réciproquement lors de l'insertion des éléments de serrage dans les logements.

12. Dispositif de fixation de tuyau souple selon l'une des revendications 1 à 6, **caractérisé** en ce que sont prévus des logements pour des corps de fixation, qui sont placés sur la partie de tuyau souple.

13. Dispositif de fixation de tuyau souple selon la revendication 12, **caractérisé** en ce que les corps de fixation sont formés par moulage sur la partie de tuyau souple.

14. Dispositif de fixation de tuyau souple selon l'une des revendications 1 à 6, **caractérisé** en ce que la partie de tuyau est reliée de manière fixe au dispositif de fixation de tuyau souple.

15. Dispositif de fixation de tuyau souple selon la revendication 14, **caractérisé** en ce que la partie de tuyau souple est collée ou encastrée par moulage par injection.

16. Pompe à galets pour une machine cardio-pulmonaire comprenant un dispositif de fixation de tuyau souple (7) selon l'une des revendications 1 à 15 précédentes.
